(19) Europäisches Patentamt
European Patent Office
Office européen des brevets

(11) EP 1 457 556 A1

(12) **EUROPEAN PATENT APPLICATION**
published in accordance with Art. 158(3) EPC

(43) Date of publication:
**15.09.2004 Bulletin 2004/38**

(21) Application number: **02790932.4**

(22) Date of filing: **27.12.2002**

(51) Int Cl.[7]: **C12N 15/09**, C12Q 1/68, G01N 33/566, A61K 45/00, A61K 39/395, A61K 48/00, A61P 43/00

(86) International application number:
**PCT/JP2002/013782**

(87) International publication number:
**WO 2003/057880 (17.07.2003 Gazette 2003/29)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **28.12.2001 JP 2001400280**

(71) Applicant: **Takeda Chemical Industries, Ltd.**
**Osaka-shi, Osaka 541-0045 (JP)**

(72) Inventors:
- **KOBAYASHI, Makoto**
  **Kobe-shi, Hyogo 651-2276 (JP)**
- **ARAI, Toshimitsu**
  **Tennoji-ku, Osaka-shi, Osaka 543-0002 (JP)**
- **MATSUMURA, Fumika,**
  **Takeda Matsushiro Residence 613**
  **Tsukuba-shi, Ibaraki 305-0035 (JP)**

(74) Representative: **Rickard, Timothy Mark Adrian**
**Takeda Patent Office,**
**11-12 Charles II Street**
**London SW1Y 4QU (GB)**

# (54) METHOD OF QUANTIFYING NUCLEIC ACID AND KIT FOR QUANTIFYING NUCLEIC ACID

(57) It is intended to provide a method of quantifying a nucleic acid and a kit for quantifying a nucleic acid. More specifically, it is intended to provide a standard comprising a synthetic polynucleotide obtained by chemical synthesis which is to be used for forming a calibration curve employed for quantifying a specific target nucleic acid in a sample, a quantification method and a quantification kit using the same, a method of diagnosing a specific disease, etc. The standard is a synthetic polynucleotide obtained by chemical synthesis. Compared with the existing standards comprising biosynthesized polynucleotides, it has an advantage that a target sequence can be precisely obtained by a convenient method. Moreover, the above-described standard suffers from little biological contamination. Therefore, it is highly safe to the environment and factors disturbing highly precise quantification can be lessened therein.

EP 1 457 556 A1

<110> Takeda Chemical Industries, Ltd.

<120> Method of Quantifying Nucleic Acid And Kit for Quantifying Nucleic Acid

<130> P02-0156

<150> JP 2001-400280
<151> 2001-12-28

<160> 10

<210> 1
<211> 2162
<212> DNA
<213> Homo Sapiens

<220>
<221> misc_feature
<222> 2063.. 2091
<223> n stands for any base

<400> 1
ggaattccgg ctataggcag aggagaatgt cagatgctca gctcggtccc ctccgcctga 60
cgctcctctc tgtctcagcc aggactggtt tctgtaagaa acagcaggag ctgtggcagc 120
ggcgaaagga agcggctgag gcgcttggaa cccgaaaagt ctcggtgctc ctggctacct 180
cgcacagcgg tgcccgcccg gccgtcagta ccatggacag cagcgctgcc cccacgaacg 240
ccagcaattg cactgatgcc ttggcgtact caagttgctc cccagcaccc agccccggtt 300
cctgggtcaa cttgtcccac ttagatggca acctgtccga cccatgcggt ccgaaccgca 360
ccaacctggg cgggagagac agcctgtgcc ctccgaccgg cagtccctcc atgatcacgg 420
ccatcacgat catggccctc tactccatcg tgtgcgtggt ggggctcttc ggaaacttcc 480
tggtcatgta tgtgattgtc agatacacca agatgaagac tgccaccaac atctacattt 540
tcaaccttgc tctggcagat gccttagcca ccagtaccct gcccttccag agtgtgaatt 600
acctaatggg aacatggcca tttggaacca tcctttgcaa gatagtgatc tccatagatt 660
actataacat gttcaccagc atattcaccc tctgcaccat gagtgttgat cgatacattg 720
cagtctgcca ccctgtcaag gccttagatt tccgtactcc ccgaaatgcc aaaattatca 780
atgtctgcaa ctggatcctc tcttcagcca ttggtcttcc tgtaatgttc atggctacaa 840
caaaatacag gcaaggttcc atagattgta cactaacatt ctctcatcca acctggtact 900
gggaaaacct cgtgaagatc tgtgttttca tcttcgcctt cattatgcca gtgctcatca 960
ttaccgtgtg ctatggactg atgatcttgc gcctcaagag tgtccgcatg ctctctggct 1020
ccaaagaaaa ggacaggaat cttcgaagga tcaccaggat ggtgctggtg gtggtggctg 1080
tgttcatcgt ctgctggact cccattcaca tttacgtcat cattaaagcc ttggttacaa 1140
tcccagaaac tacgttccag actgtttctt ggcacttctg cattgctcta ggttacacaa 1200
acagctgcct caacccagtc ctttatgcat ttctggatga aaacttcaaa cgatgcttca 1260
gagagttctg tatcccaacc tcttccaaca ttgagcaaca aaactccact cgaattcgtc 1320
agaacactag agaccacccc tccacggcca atacagtgga tagaactaat catcagctag 1380
aaaatctgga agcagaaact gctccgttgc cctaacaggg tctcatgcca ttccgacctt 1440
caccaagctt agaagccacc atgtatgtgg aagcaggttg cttcaagaat gtgtaggagg 1500
ctctaattct ctaggaaagt gcctactttt aggtcatcca acctctttcc tctctggcca 1560
ctctgctctg cacattagag ggacagccaa aagtaagtgg agcatttgga aggaaaggaa 1620
tataccacac cgaggagtcc agtttgtgca agacacccag tggaaccaaa acccatcgtg 1680
gtatgtgaat tgaagtcatc ataaaaggtg acccttctgt ctgtaagatt ttattttcaa 1740
gcaaatattt atgacctcaa caaagaagaa ccatcttttg ttaagttcac cgtagtaaca 1800
cataaagtaa atgctacctc tgatcaaagc accttgaatg gaaggtccga gtctttttag 1860
tgtttttgca agggaatgaa tccattattc tattttagac ttttaacttc aacttaaaat 1920
tagcatctgg ctaaggcatc attttcacct ccatttcttg gttttgtatt gtttaaaaaa 1980
aataacatct ctttcatcta gctccataat tgcaagggaa gagattagca tgaaaggtaa 2040
tctgaaacac agtcatgtgt canctgtaga aaggttgatt ctcatgcact ncaaatactt 2100
ccaaagagtc atcatggggg atttttcatt cttaggcttt cagtggtttg ttcctggaat 2160
tc 2162

<210> 2
<211> 25
<212> DNA
<213> Artificial Sequence

<220>
<223> Primer N917F

<400> 2
ccttggttac aatcccagaa actac 25

<210> 3
<211> 24
<212> DNA
<213> Artificial Sequence

<220>
<223> Primer N-998R

<400> 3
aggcagctgt ttgtgtaacc taga 24

<210> 4
<211> 29
<212> DNA
<213> Artificial Sequence

<220>
<221> misc_binding
<223> Probe N-945T, labeled 5'-terminal with FAM and 3'-terminal with TAMRA

<400> 4
ccagactgtt tcttggcact tctgcattg 29

<210> 5
<211> 24
<212> DNA
<213> Artificial Sequence

<220>
<223> Primer

<400> 5
ccttcgcaag acccttcctc tata 24

<210> 6
<211> 23
<212> DNA
<213> Artificial Sequence

<220>
<223> Primer

<400> 6
gtagctgtcg gtgtagtcct cgt 23

<210> 7
<211> 20
<212> DNA
<213> Artificial Sequence

<220>
<223> Primer

<400> 7
ttacggcgag ttctgttagg 20

<210> 8
<211> 20
<212> DNA
<213> Artificial Sequence

<220>
<223> Primer

<400> 8

```
catgtgcctg agaaataggc                                                 20

<210> 9
<211> 20
<212> DNA
<213> Artificial Sequence

<220>
<223> Primer

<400> 9
aaaagagctg tcctgacagc                                                 20

<210> 10
<211> 20
<212> DNA
<213> Artificial Sequence

<220>
<223> Primer

<400> 10
tcctcctgca tcaattgtgt                                                 20
```

## Description

### Background Art

The present invention relates to a standard preparation that is used to draw up a calibration curve used to quantify a specific target nucleic acid in a sample, a method for quantifying a nucleic acid thereby, or a quantitative kit. The present invention also relates to a method for diagnosing a specific disease by using the nucleic acid quantitative method or quantitative kit, or a medicine comprising the specified gene DNA or the gene product.

Since it is possible to detect a small amount of a target nucleic acid after amplifying it exponentially by using the PCR (polymerase chain reaction) method, the PCR method is largely used in the fields of expression analysis of genes, diagnosis of disease, detection of food containing recombinant genes or determining the presence of recombinant genes in natural foods. The PCR method is, for example, disclosed in US Patent 4,683,195, US Patent 4,683,202 and US Patent 4,965,188 and others in detail. The general PCR method can easily detect a small amount of a target nucleic acid by reacting the sample that may contain the target nucleic acid with an amplification reagent comprising a pair of oligonucleotide primer corresponding to the target nucleic acid, reaction substrate (deoxynucleotide triphosphate), and DNA polymerase and the like, and exponentially amplifying the target nucleic acid.

Additionally, the TaqMan method is known, which uses a fluorescent probe and the like in PCR method as a method for quantifying a specific mRNA with high accuracy. For example, Patent Kokai 2001-204483 discloses a TaqMan method to quantify hTERT mRNA.

Further, a technique using an internal standard has been developed to improve the accuracy in quantifying a small amount of a target nucleic acid, for example, as disclosed by the publication of Patent Kokai H11-123095. This publication discloses a method for quantifying a target nucleic acid in which a plasmid including a DNA sequence that behaves as an internal standard is used with an amplification reagent, and the cRNA produced by this plasmid is used as an internal standard.

By the above method, it is possible to quantify a target nucleic acid more accurately than with a method that does not use an internal standard. However, since the RNA as an internal standard is obtained from a plasmid in a reactor by this method, a specific and constant amount of RNA cannot always be prepared depending on the storage condition of the reagent, the reaction condition, and other factors. Furthermore, a standard preparation produced by the conventional enzyme or organism is complex and is often difficult to prepare. In order to prepare the standard preparation by the conventional method, it is necessary to extract genome DNA or RNA or mRNA from cells, organs or virus of the desired species on the basis of the sequence information and to enzymatically or biologically synthesize the segment of the desired gene or DNA or RNA having those characteristics. Moreover, the species used as materials include some that are difficult to obtain or have strong pathogens. Furthermore, in the preparation of the standard preparation, enzymes extracted from organisms or the organisms per se are used, which lead to introduction of biological contaminants that prevent highly accurate quantification.

Therefore, if there were provided a standard preparation that does not have the above disadvantages, i.e., a standard preparation obtainable by a convenient method, and having no biological contaminant, or a method capable of quantifying a target nucleic acid with a high accuracy using the standard preparation, then it would be useful in the fields of identification of disease genes, diagnosis of specific diseases, treatment of diseases, or the like.

### Disclosure of the Invention

Therefore, the present invention provides a standard preparation, a method for quantifying the nucleic acid, a kit for quantifying nucleic acid, a method for diagnosis of a specific diseases, and the like, as described below.

(1) A standard preparation useful to quantify or detect a specific target nucleic acid in a sample, comprising a synthetic polynucleotide obtained by chemical synthesis.
(2) The standard preparation of (1), wherein the synthetic polynucleotide is RNA, DNA or a modification thereof.
(3) The standard preparation of (1), wherein the synthetic polynucleotide is RNA or DNA.
(4) The standard preparation of (3), which is a sense strand if the synthetic polynucleotide is RNA, or which is an antisense strand if the synthetic polynucleotide is DNA.
(5) The standard preparation of (1), wherein the synthetic polynucleotide is a synthesized part of the target nucleic acid, and the number of nucleotides is between 60 and 200.
(6) A kit for quantifying nucleic acid comprising the standard preparation of any one of claims (1) to (5).
(7) A kit for quantifying nucleic acid comprising the standard preparation of any one of claims (1) to (5) and at least one pair of primers.
(8) The kit of (7), additionally comprising a fluorescent probe or a phosphorylated probe.
(9) The kit of (8), additionally comprising a DNA polymerase.

(10) The kit of (9), additionally comprising a reverse transferase.

(11) A kit for quantifying nucleic acid to quantify multiple target nucleic acids, wherein an amplification reagent comprising a pair of primers corresponding to the target nucleic acid is loaded with at each reaction site of a reactor having multiple reaction sites, and an amplification reagent comprising the standard preparation of any one of claims 1 to 5 and a pair of primers corresponding to the standard preparation is loaded at a reaction site which is not loaded with a pair of primers corresponding to the target nucleic acid.

(12) The kit for quantifying nucleic acid of (11), which is used to diagnose the specific disease, and wherein the multiple target nucleic acids are DNA or mRNA related to the specific disease.

(13) The kit for quantifying nucleic acid of (11), which is used to detect recombinant DNA in food, and wherein the multiple target nucleic acids are recombinant DNA contained in genetically-modified food.

(14) A method for quantifying a specific target nucleic acid in a sample, which comprises adding an amplification reagents comprising at least one pair of primers corresponding to a target nucleic acid to the sample, adding to a chemically synthesized polynucleotide as a standard preparation, an amplification reagent comprising a pair of primers corresponding to the synthesized polynucleotide, carrying out each amplification reaction, measuring the amounts of the amplified standard preparation and the amplified target nucleic acid, and calculating the amount of the target nucleic acid before amplification according to the information obtained by the measurements.

(15) The method of (14), wherein the synthetic polynucleotide is RNA, DNA or a modification thereof.

(16) The method of (14), wherein the synthetic polynucleotide is RNA.

(17) The method of (16), wherein the synthetic polynucleotide is a sense strand.

(18) The method of any one of (14) to (17), wherein the synthetic polynucleotide is a synthesized part of the target nucleic acid, and the number of nucleotides is between 60 and 200.

(19) The method of any one of (14) to (18), wherein the sample is an mRNA sample of human or other animal origins.

(20) The method of (19), wherein the amplification reagent additionally comprises a fluorescence probe or a phosphorylated probe.

(21) The method of (20), wherein the amplification reagent additionally comprises a DNA polymerase.

(22) The method of (21), wherein the amplification reagent additionally comprises a reverse transferase.

(23) The method of any one of (20) to (22), wherein (1) a probe portion of a fluorescence probe or a phosphorylated probe contained in the amplification reagent comprising at least one pair of primers corresponding to a target nucleic acid, is a probe consisting of the nucleic acid of the region between the pair of primers in the target nucleic acid, and (2) a portion of the a fluorescence probe or a phosphorylated probe contained in the amplification reagent comprising the pair of primers corresponding to the synthetic polynucleotide, is a probe consisting of the nucleic acid of a region between the pair of primers in the synthetic polynucleotide.

(24) The method of (23) to measure the amount of the amplified standard preparation and the amount of the amplified target nucleic acid using the fluorescence intensity of the fluorescent substance released from the fluorescence probe or the phosphorylated probe by DNA polymerase or the amount of phosphate group as an index.

(25) A method for analyzing SNPs which uses the kit of (11) or the method of (14).

(26) A method for diagnosing a specific disease, which uses the kit of (12) or the method of (14).

(27) A method for determining if food contains recombinant gene DNA or not, which uses the kit of (13) or the method of (14).

(28) A medicine which is specified by the kit of (12) or the method of (26), which comprises a gene DNA in which expression is distinctively increased or reduced in a certain cell or tissue, or a gene product thereof, or an agonist, antagonist, or antibody against the gene product.

The present invention will be described in detail below.

## The Best Mode for Carrying Out the Invention

### (Standard Preparation)

The standard preparation of the present invention is used to quantify or detect a specific target nucleic acid in a sample and is characterized by comprising a synthetic polynucleotide obtained by chemical synthesis. The "standard preparation" means a standard preparation used to draw up a calibration curve used to calculate the amount of a target nucleic acid before amplification or used to detect a specific nucleic acid sequence such as an SNP. The methods of drawing the calibration curve and calculating the amount of target nucleic acid before amplification are described later.

The standard preparation used in the present invention is a synthetic polynucleotide, and is preferably a single strand or double strand RNA obtained by chemical synthesis, a single strand or double strand DNA, or a modification thereof. The "modification" means those having a portion chemically modified, for example: (1) those having a chemically modified purine ring and/or pyrimidine ring (for example, those having methylated purine or pyrimidine ring or acylated purine or pyrimidine ring), or those comprising other heterocyclic ring, (2) those having a chemically modified sugar portion(for example, those wherein one or more hydroxyl groups are substituted with halogen or aliphatic groups, or substituted with a functional group such as ether or amine), (3) those with biotinylation, (4) those with FITC conjugation, (5) those modified with digoxigenin, (6) those with phosphorylation, (7) those modified with peroxidase, (8) those modified with alkaliphosphatase, (9) those modified with luciferase.

Since it is possible to obtain entirely by chemical synthesis the needed amount of the desired polynucleotide having a single chemical structure, a chemical synthesized polynucleotide is used in the present invention.

The standard preparation of the present invention is, preferably, a single strand obtained by chemical synthesis, more preferably, a sense strand obtained by chemical synthesis. Further, in the case of using a single chemically synthesized strand, an antisense strand is preferable. Because using a single strand rather than a double strand allows the standard to more closely resemble the condition of the nucleic acid being quantified, the accuracy of the quantification is improved.

The synthesized polynucleotide of the present invention may be a synthesized polynucleotide having the same nucleic acid sequence as the target nucleic acid or a portion thereof, and having the similar sequence of the standard preparation obtained by conventional biosynthesis. Such standard preparations include, for example, 18S Ribosomal RNA, Acidic Ribosomal Protein, β-actin, Cyclophilin, Glyceraldehyde-3-phosphate dehydrogenase, Phosphoglycerokinase, β2-microglobulin, β-Glucuronidase, Hypoxanthine Ribosyl Transferase, Transcription Factor IID/TATA Binding Factor, Transferrin Receptor, and the like.

It is easy for a skilled artisan to synthesize a polynucleotide for the standard preparation of the present invention by chemical synthesis on the basis of the known sequence. Synthesis methods of known polynucleotides include, for example, the phosphoamidide method, H-phosphate method, and other known methods.

The phosphoamidide method is disclosed, for example, in Wu, T., Ogilvie, K. K., and Pon, R. T. (1989) "Prevention of chain cleavage in the chemical synthesis of 2'-O-silylated oligoribonucleotides" Nucl. Acids Res. 17, 3501-3517; Stawinski, J., Stromberg, R., Thelin, M., and Westman, E. (1988) "Studies on the t-butyldimethyl-silyl group as 2'-O-protection in oligoribonucleotide synthesis via the H-phosphonate approach" Nucl. Acids Res. 16, 9285-9288; Scaringe, SA. A., Franklyn, C., and Usman, N. (1990) "Chemical synthesis of biologically active oligoribonucleotides using b-cyanoethyl protected ribonucleoside phosphoramidites" Nucl. Acids Res. 18, 5433-5441; Chaix, C., Molko, D. and Teoule, R. (1989) "The use of labile base protecting groups in oligoribonucleotide synthesis" Tetrahedron Lett. 30, 71-74; Gasparutto, D., Livache, T., Bazin, H., Duplaa, A. M., Guy, A., Khorlin, A., Molko, D., Roget,A., and Teoule, R. (1992) "Chemical synthesis of a biologically active natural tRNA with its minor bases" Nucleic Acids Res. 20, 5159-5166; Vinayak, R., Anderson, P. McCollum, C., and Hampel, A. (1992) "Chemical synthesis of RNA using fast oligonucleotide deprotection chemistry" Tetrahedron Lett. 31, 7269-7272; and the like. Further, H-phosphate method is, for example, disclosed in Garegg, P. J., Regberg, T., Stawinski, J. and Stromberg, R. (1985). Formation of internucleotidic bonds via phosphonate intermediates. Chem. Scripta 25, 280-282 ; Garegg, P. J., Regberg, T., Stawinski, J. and Stromberg, R. (1986). Nucleoside hydrogenphosphonates in oligonucleotide synthesis. Chem. Scripta 26, 59-62 ; Garegg, P. J., Lidh, I., Regberg, T., Stawinski, J. and Stromberg, R. (1986) ; Nucleoside H-phosphonates. III. Chemical synthesis of oligodeoxyribonucleotides by the hydrogenphosphonate approach. Tetrahedron Lett. 27, 4051-4054 ; Froehler, B. C., Ng, P. G., and Matteucci, M. D. (1986). Synthesis of DNA via deoxynucleoside H-phosphonate intermediates. Nucleic Acids. Res. 14, 5399-5407 ; Froehler, B. C., and Matteucci, M. D. (1986). Nucleoside H-phosphonates : Valuable intermediates in the synthesis of oligonucleotides. Tetrahedron Lett. 27, 469-472 and the like.

In addition, a synthesized polynucleotide used in the present invention would preferably be a short one in view of the amplification efficiency, for example, having 60 to 150mers, more preferably, 60 to 100mers. The number of the nucleotides of the synthesized polynucleotide in the present invention is not specifically limited, and may be in the range of 60 to 200, preferably, 60 to 100.

**(Nucleic Acid Quantitative Kit)**

The kit for quantifying nucleic acid used in the present invention comprises a synthesized polynucleotide as the above standard preparation, and may further comprise at least a pair of primers corresponding to a target nucleic acid, a probe corresponding to a target nucleic acid, DNA polymerase, buffer, and the like. These kits may comprise, for example, a reagent catalyzing a synthesis of primer extension products, nucleoside triphosphate of a substrate, and implements used as indicators (for example, if the indicator is biotin, an adipin-enzyme conjugate, the substrate of the enzyme, and chromogen), buffer suitable for the reaction of PCR or hybridization, if necessary.

The phrase "pair of primers corresponding to a target nucleic acid" used in the present invention means a pair of primers consisting of a first primer which is complementary or substantially complementary to one strand of the exon region of a sequence of a target gene (or a gene sequence encoding a target mRNA) and a second primer which is complementary or substantially complementary to the exon region of the target gene sequence on other strand. The exon region between the first primer and the second primer is amplified.

In the present invention, a target nucleic acid is amplified using at least a pair of these primers.

Those skilled in the art can easily design pairs of primers to amplify a target nucleic acid or pairs of primers to amplify a standard preparation on the basis of the target nucleic acid (for example, Genome Res. 1996 Oct; 6 (10) : 986-94 reference).

For example, if hMOR1 cDNA is selected as a target nucleic acid, a complementary sequence to the $1129^{th}$ to $1210^{th}$ base of hMOR1 cDNA (SEQ ID NO:1) can be used as a standard preparation by chemical synthesis. In this case, 5'-CCTTGGTTACAATCCCAGAAACTAC-3' (SEQ ID NO: 2) is used as an upstream primer and 5'-AGGCAGCTGTTTGTGTAACCTAGA-3' (SEQ ID NO:3) as a downstream primer.

It is easy for those skilled in the art to design a probe corresponding to a synthesized polynucleotide used in the present invention or a probe corresponding to a synthesized probe which is a standard preparation according to a target sequence (for example, Genome Res. 1996 Oct ; 6 (10) : 986-94 reference). For example, a probe corresponding to a target nucleic acid is a probe consisting of a nucleic acid of the region between the first primer and the second primer in the target nucleic acid. A primer corresponding to a synthetic polynucleotide is a probe consisting of a nucleic acid of the region between the first primer and the second primer. An adequate oligonucleotide probe used in the present invention would preferably have about 15 to about 50 nucleotides, and more preferably about 25 to about 35 nucleotides. An oligonucleotide probe can be labeled by incorporating chemical substances and the like that are detectable by biochemical, immunochemical, or chemical means. Some useful indicators include radioisotopes of $P^{32}$ or the like; fluorescent substances such as fluorescamine, fluorescein isothio- cyanate; luminescent substances such as luminal or luciferin; enzymes such as β-galactosidase, peroxidase, alkaliphosphatase; biotin, antibodies and the like. In particular, preferred are fluorescent probes labeled by fluorescent substances or phosphated probes labeled by $P^{32}$.

The DNA polymerases used in the present invention are listed as thermostable DNA polymerases having reverse transferase activity and 5'→3' exonuclease activity, for example, Tth DNA polymerase and the like.

In the present invention, known or commercially available buffers may be used (for example, buffer produced by PEB isosystems; Genome Res. 1996 Oct. ; 6(10) : 986-94 reference ).

In addition, the amount of each reagent that is comprised in the quantitative kit is appropriately determined according to the amount of a sample or kind of a target nucleic acid.

The more preferable embodiment of the present invention provides a kit for quantifying nucleic acid to quantify multiple target nucleic acids, wherein each amplification reagent comprising a pair of primers corresponding to a target nucleic acid is loaded at each of multiple reaction sites (preferably, each reaction site of a reactor having multiple reaction sites), and an amplification reagent comprising the standard preparation of any one of claims 1 to 4 and a pair of primers corresponding to the standard preparation is loaded at a reaction site which is not loaded with a pair of primers corresponding to the target nucleic acid. It is possible to detect the presence or absence of multiple target nucleic acids or the amount thereof in a sample in a single procedure by using this quantitative kit.

The multiple reaction sites are not specifically limited as far as there are two or more reaction sites, generally, 2 to tens of thousands, preferably, 2 to 1,000, more preferably 10 to 800, even more preferably, 10 to 300 reaction sites.

In this embodiment, a reactor having multiple reaction sites is used. Each reaction of a sample that may comprise a target nucleic acid with each amplification reagent, and each of standard preparations consisting of a synthesized nucleotide set up in successive prescribed concentrations with each amplification reagent are carried out at each reaction site. The concentration of the standard preparation is not particularly limited, but preferably is in the range of $10^1$ copies to $10^7$ copies or more in each container. Each amplification reagent comprises a pair of primers capable of amplifying a target nucleic acid, or the synthesized polynucleotide, respectively. The reactor used herein is not particularly limited by the configuration and structure of the reactor as long as there are two or more reaction sites so that a sample and each amplifying reagent can react in a single procedure. Preferably, the reactors used in the present invention include, for example, a plate having multiple wells, a reactor having multiple slide glasses, a reactor having multiple test tubes and the like. In view of the experiment space, operability, or the like, preferably, a plate having multiple wells can be used. The numbers of the wells of these plates are determined by the numbers of the reagents. Preferred are commercial 96-well plates and 384-well plates. However, any reactors that have the desired number of reaction sites according to the numbers of reagents can be used.

Since the number of amplification reagents is regulated only according to the number of target nucleic acids, there is no limit to their number: for example, one could have a quantitative kit comprising a standard preparation consisting of 10 to 800 kinds of amplification reagents and synthetic oligonucleotides for the targets provided. In another embodiment, a kit comprising the standard preparation consisting of 10 to 300 kinds of amplification reagents and a synthetic oligonucleotide for the target is given. If there are many amplification reagents, it is possible to divide the different sets of amplification reagents onto multiple plates (for example, on 2 to 10 plates) and carry out quantitative reactions several times.

**(Method for Quantifying Nucleic Acids)**

The method for quantifying nucleic acids is described below.

According to a method for quantifying a specific target nucleic acid of the present invention, amplification reagents comprising at least one pair of primers corresponding to each target nucleic acid are added to the sample. An amplification reagent comprising a pair of primers corresponding to the synthetic polynucleotide is added to the chemical synthetic polynucleotide as a standard preparation. Amplification reactions are carried out on both respectively, amounts of the standard preparation and the amplified target nucleic acid are measured, and the amount of the target nucleic acid before amplification is calculated according to those measurements.

**[Sample and Target nucleic acid]**

First, samples of this invention may comprise a target nucleic acid, and are not particularly limited. The samples of this invention include, for example, a tissue or mRNA sample derived from cultivated cells of human or other mammal (e.g. guinea pig, rat, mouse, rabbit, sheep, swine, bovine, feline, canine, monkey, etc.). Examples of such tissues used as samples might include: brain, brain regions (e.g., olfactory bulb, amygdaloid nucleus, cerebral basal bulb, hippocampus, thalamus, hypothalamus, substhanlamic nucleus, cerebral cortex, medulla oblongata, cerebellum, occipital lobe, frontal lobe, temporal lobe, putamen, caudate nucleus, corpus callosum, substantia nigra), spinal cord, hypophysis, stomach, pancreas, kidney, liver, gonad, thyroid, gallbladder, bone marrow, adrenal gland, skin, muscle, lung, gastrointestinal tract (e.g., large intestine and small intestine), blood vessel, heart, thymus, spleen, submandibular gland, peripheral blood, peripheral hemocyte, prostate, testis, ovary, placenta, uterus, bone, joint, skeletal muscle, etc. By quantifying the target mRNA contained in the mRNA sample, it is possible to analyze the expression level of the target gene at the site where the mRNA is obtained.

Further, use of an mRNA sample derived from a patient having a specific disease allows easy characterization of a gene related to the disease (for example, GPCR gene). Particularly, in the case of identifying a G protein coupled receptor, tyrosine phosphatase receptor, ion channel, or other such genes associated with multiple-gene diseases such as a cancer, in which multiple genes seem to be related, the present invention is useful to characterize the related gene or protein. This is because the expression level of each gene can be measured by one quantitative operation.

By using the synthetic polynucleotide of the present invention as standard preparation for quantification or detection of specific target nucleic acid in a sample as described above, it is possible to (1) identify genes of which expression is characteristically increased or reduced in certain cells or tissues by quantitatively analyzing each expression level of multiple genes at the same time, and (2) identify a certain gene in a given gene family of which expression is characteristically increased or reduced in certain cells or tissues, by analyzing the expression of multiple genes belonging to that specific gene family at the same time and calculating the absolute value of the expression level of the gene.

The "multiple genes" are meant to signify two or more genes. There is no specific upper bound, within limits of feasibility, but the range is commonly two to several tens of thousands of genes, or preferably, two to 1,000 genes, or more preferably, 10 to 800 genes, or even more preferably, 10 to 300 genes.

"The expression level of a gene is characteristically increased or reduced" is meant, compared with the expression of a gene in normal cells or tissues, to refer to a physiologically significant difference in expression, whether small or large. The target nucleic acid family in the present invention is not specified, but for example, it could be selected from the gene family concerning the G protein coupled receptor gene family, tyrosine phosphatase receptor gene family, ion channel gene family, or transcription factor, transporter, protein kinase, protein phosphatase, protease, heat shack protein, ATPase or DNA-binding protein gene family or the like.

The calculation of the level of gene expression or absolute value of gene expression can be carried out according to the quantitative method using a target mRNA as described below.

Also, according to the present invention, use of DNA included in recombinant gene foods as the target nucleic acid may allow detection of recombinant gene foods or presence of recombinant genes in natural foods obtained without using recombinant gene techniques.

Currently known recombinant gene foods include soybean, potato, corn, tomato, papaya. Further, the recombinant genes in those foods and pairs of primers to detect them, general quantitative methods or the like are known. For example, they are disclosed in "JAS Analysis Handbook recombinant gene foods detection/ analysis manual quantitative PCR" (H13. April, published by Tokyo IAA Center for Food Quality, Labeling and Consumer Services).

For example, in the detection of corn CB351, a known pair of primers consists of 5'-CCT TCG CAA GAC CCT TCC TCT ATA-3' (SEQ ID NO:5) and 5'-GTA GCT GTC GGT GTA GTC CTC GT-3' (SEQ ID NO:6). For the detection of papaya 55-1, a known pair of primers consists of 5'-TTA CGG CGA GTT CTG TTA GG-3' (SEQ ID NO:7) and 5'-CAT GTG CCT GAG AAA TAG GC-3'(SEQ ID NO:8). For the detection of potato New Leaf Y, a known pair of primers consists of 5'-AAA AGA GCT GTC CTG ACA GC-3' (SEQ ID NO:9) and 5'-TCC TCC TGC ATC AAT TGT GT-3'(SEQ ID NO:10).

In another embodiment of the present invention, the target nucleic acid may be a G protein coupled receptor, tyrosine phosphatase receptor or ion channel coding gene DNA, or mRNA thereof. In this case, by reacting each amplification reagent comprising a pair of primers corresponding to the mRNA of a gene belonging to a family such as target G protein coupled receptor, tyrosine phosphatase receptor, ion channel receptor or the like with an mRNA sample at each reaction site of the reactor, carrying out amplification reactions, and quantifying mRNA amplified products, it is possible to measure the expression level of a gene belonging to gene families such as the G protein coupled receptor, the tyrosine phosphatase receptor, the ion channel receptor gene family, or the like.

In another embodiment of the present invention, by completely preparing all pairs of primers corresponding to the mRNA of a gene belonging to given gene family such as all of the known G protein coupled receptor, tyrosine phosphatase receptor, ion channel receptor gene families, and the like, and reacting amplification reagents comprising those pairs of primers, respectively at each reaction site, it is possible to measure the degree of production of each mRNA of the gene belonging to gene families such as the G protein coupled receptor, tyrosine phosphatase receptor, ion channel receptor gene families, and the like in an mRNA sample at the same time. Of course, it is also possible to carry out quantitative reactions several times by appropriately dividing the different sets of amplification reagents onto multiple plates when necessary.

In another embodiment of the present invention, by using a group of pairs of primers corresponding to the mRNA of a gene belonging to a family such as the G protein coupled receptor, tyrosine phosphatase, or the ion channel receptor gene family, with one quantitative manipulation, it is possible to characterize the gene belonging to gene families such as the G protein coupled receptor, tyrosine phosphatase receptor, or ion channel receptor gene family highly expressed in the genes belonging to the gene families that include the group of G protein coupled receptors, tyrosine phosphatase receptors, ion channel receptors, and the like.

In addition, the following are currently known as G protein coupled receptors:

(1) Acetylcholine receptors: $M_1$; $M_2$; $M_3$; $M_4$; $M_5$
(2) Adenosine receptors: $A_1$; $A_{2A}$; $A_{2B}$; $A_3$
(3) Adrenoceptors: α1A; α1B; α1D; α2A; α2B; α2C; β1; β2; β3
(4) Angiotensin receptors: AT1; AT2
(5) Bombesin receptors: BB1; BB2; bb3
(6) Bradykinin receptors: $B_1$; $B_2$
(7) Calcitonin, Ainilin, CGRP, and Adrenomedullin receptors:
(8) Cannabinoid receptors: CB1;CB2
(9) Chemokine receptors: CCR1; CCR2; CCR3; CCR4; CCR5; CCR6; CCR7; CCR8; CCR9; CCR10; CXCR1; CXCR2; CXCR3; CXCR4; CXCR5; $CX_3CR1$; XCR1;
(10) Cheniotactic receptors : $C3_a$; $C5_a$; fMLP
(11) Cholecystokinin and Gastrin receptors: $CCK_1$; $CCK_2$
(12) Corticotropin-releasing factor receptors: $CRF_1$; $CRF_2$
(13) Dopamine receptors: D1; D2; D3; D4; D5
(14) Endothelin receptors: $ET_A$; $ET_B$
(15) Galanin receptors: GAL 1; GAL2; GAL3
(16) Glutamate receptors: $mgl_1$; $mgl_2$; $mgl_3$; $mgl_4$; $mgl_5$; $mgl_6$; $mgl_7$; $mgl_8$
(17) Glycoprotein hormone receptors: FSH; LSH; TSH
(18) Histamine receptors: $H_1$; $H_2$; $H_3$; $H_4$
(19) 5-HT receptors: 5-$HT_{1A}$; 5-$HT_{1B}$; 5-$HT_{1D}$; 5-$ht_{1B}$; 5-$ht_{1F}$; 5-$HT_{2A}$; 5-$HT_{2F}$; 5-$HT_{2C}$; 5-$HT_3$; 5-$HT_4$; 5-$ht_{5A}$; 5-$ht_{5B}$; 5-$HT_6$; 5-$HT_7$
(20) Leukotriene receptors: BLT; $CysLT_1$; $CysLT_2$
(21) Lysophospholipid receptors: edg1; edg2; edg3; edg4
(22) Melanocorlin receptors: $MC_1$; $MC_2$; $MC_3$; $MC_4$; $MC_5$
(23) Melatonin receptors: $MT_1$; $MT_2$; $MT_3$
(24) Neuropeptide Y receptors: $Y_1$; $Y_2$;$Y_4$; $Y_5$; $Y_6$
(25) Neurotension receptors: NTS1; NTS2
(26) Opioids: DOP; KOP; MOP; NOP
(27) P2Y receptors: $P2Y_1$; $P2Y_2$; $P2Y_4$; $P2Y_6$; $P2Y_{11}$; $P2Y_{12}$
(28) Peroxisome proliferators: PPAR-α; PPAR-β; PPAR-γ
(29) Prostanoid receptors: DP; FP; IP; TP; $EP_1$; $EP_2$; $EP_3$; $EP_4$
(30) Protease-activated receptors: PAR1; PAR2; PAR3; PAR4
(31) Somatostatin receptors: $sst_1$; $sst_2$; $sst_3$; $sst_4$; $sst_5$
(32) Tachykinin receptors: $NK_1$; $NK_2$; $NK_3$

(33) Thyrotropin-releasing hormone receptors: $TRH_1$; $TRH_2$

(34) Urotensin-II receptor:

(35) Vasoactivate intestinal peptide or pituitary adenylate cyclase activating peptide receptors: $VPAC_1$; $VPAC_2$; $PAC_1$

(36) Vasopressin or Oxytocin receptors: $V_{1a}$; $V_{1b}$; $V_2$; OT

Additionally, the following are known as genes belonging to families such as tyrosine phosphatase receptor, ion channel receptor, or the like.

(37) Ion channel: $Na^+$ channels (type I; type II/type IIA; type III; SCL/NaG; PN1; NaCh6; NaDRG; SkM1/μI, or SkM2), $K^+$ channels (kv; EAG; KQT; IRK; ROMK; GIRK; $K_{ATP}$ or the like ), $Ca^{2+}$ channels (α1G; α1E; α1S; α1C; α1D; α1B; α1A; IP3; ryanodine receptor or the like ), $Cl^-$ channels ($GABA_A$; $GABA_C$; glycine receptors; C1C0; C1C1; CFTR or the like), non-selective cation channels (nAChR; 5-$HT_3$; NMDA; AMPA; $P_{2X}$ATP; CNG, or the like) or the like.

(38) Tyrosine phosphatase receptors: insulin receptor; EGF receptor; or the like.

The nucleic acid quantitative method used in the present invention is capable of using several kinds of applications other than function analysis of human GPCR, SNP analysis, or determination of recombinant gene foods. For example, it is possible to diagnose specific diseases by using sets of multiple amplification reagents comprising a pair of primers detecting mRNA produced by the known disease gene. Because the present invention is capable of accurately measuring the expression level of each gene, it has an advantage in providing diagnoses more accurately than the known method.

**[Amplification of Nucleic Acids]**

In the quantitative method of the present invention, a target nucleic acid that may be contained in a sample is amplified by using amplifying reagents consisting of a pair of primers corresponding to the target nucleic acid. In the preferred embodiment of the present invention, amplification of a target nucleic acid may be carried out by the known polymerase chain reaction (PCR) (see US Patent No. 4683195; US Patent No. 4683202; US Patent No. 1965188 and the others).

When the target is an mRNA, the amplification of the target mRNA may be carried out, for example, by first using viral reverse transcriptase to obtain a cDNA by reverse transcription of the target mRNA, and then amplifying the obtained cDNA. In a more preferred embodiment of the present invention, the amplification of mRNA is carried out by using reverse transferase-polymerase chain reaction (RT-PCR)(US Patent No. 5310652; US Patent No. 5322770; US Patent No. 5561058; US Patent No.5641864; US Patent No.5693517)

In the present invention, many mRNA amplification methods can be used in addition to the said polymerase chain reaction. These other amplification methods include, for example, the chain substitution assay method (US Patent No. 5455166 and other, reference), the transcription amplifying system (TAS) (see US Patent No. 5437990; No. 5409818; No. 5399491 and others), and the self-sustained sequence replication system (3SR) (WO92/08800 and others, see references), among others.

One skilled in the art would easily set the conditions of these amplifying reactions by varying the kinds of reagents used.

**[Quantification of Target Nucleic Acid]**

In the next step of the quantification method of the present invention, the amount of amplified nucleic acid product created in the previous step is quantified. The quantification of this amplified product is preferably carried out by a method using a probe. According to the preferred embodiment, the above method should be one using a probe labeled with fluorescent substance.

According to the more preferred embodiment of the present invention, the quantification of target mRNA is carried out by the "TaqMan method" or "5' nuclease assay method" (see Proc. Natl. Acad. Sci. USA, vol.88, p7276-7280 (1991); US Patent No. 5210015; No. 5487972; No. 5804375; No.5804375 and others). However, if necessary, the SYBER Green method, or some hybridization method may be used. In the TaqMan assay method, a probe labeled at the 5' terminus is used. Also, this probe may be modified at the 3' terminus to prevent the probe from working as a primer to synthesize DNA. Examples of this modification could include the addition of a phosphate group or some fluorescent substance to the 3' terminus. Amplification of the target mRNA may be carried out by using a DNA polymerase having 5'→3' exonuclease activity, for example, Tth DNA polymerase. Any probes that hybridize downstream from the abovementioned primer on the target mRNA are removed by the 5'→3' exonuclease activity of DNA polymerase during the amplification reaction. During each new amplification step of the target region, the probe is removed and the labeled substance (for example, phosphate group or fluorescent substance), with which the probe was modified, is released. By quantifying the amount of this released labeling substance, the amount of target mRNA produced can be indirectly measured.

Known methods are used to detect the released target substance quantitatively. In the preferred method, the said probe is labeled at the 5' and 3' terminus by two fluorescent substances, and each substance has the ability to suppress the fluorescence of the other substance. While this probe is hybridized to template DNA, the fluorescence emitted by the two substances is suppressed by their reciprocal activity upon each other. However, when the probe is removed by the 5'-3' exonuclease activity of the DNA polymerase, these substances will begin to fluoresce. This fluorescence is increased according to progress of the amplification reaction, and this increase in fluorescence is monitored.

**[Drawing up the Calibration Curve and Calculating the Amount of target nucleic acid before Amplification]**

The present invention provides prescribed amount of a synthesized polynucleotide as astandard preparation. Thus, a sample comprising a target nucleic acid can be quantified based on the "calibration curve" drawn up after amplification of the standard preparation.

A way of drawing up this calibration curve is disclosed, for example, in the publication of Japanese Patent No. H11-123095. To draw up the calibration curve, the amount of polynucleotide as the standard preparation produced in polymerase chain reaction is plotted as against varied known amounts of RNA present before amplification. In order to ensure a high level of accuracy, the calibration curve is drawn up by carrying out the amplification reaction using a series of dilutions that gradually change the concentration of the amplification reaction mixture. This calibration curve is drawn up by plotting the amount of internal standard preparation or the target nucleic acid amplified over a given number of amplification cycles.

The amount of the target nucleic acid before amplification is determined by comparing the amount of the amplified target nucleic acid with the above calibration curve. The standard preparation and a set of dilution series of target nucleic acid is amplified at another reaction site under the same conditions; the reaction is stopped at the exponential stage of amplification; and the amount of target nucleic acid present in this sample before the amplification is determined to be extrapolated according to the calibration curve drawn up by using the standard preparation.

**[Method for Diagnosing a Disease and Medicine for Treating the Disease]**

According to the above method (23) of the present invention, gene expression analysis may be carried out using mRNA sample derived from a patient. This expression analysis provides information that can be used to diagnose the patient by detecting the characteristic expression of a specific disease-related gene. In the present invention, by analyzing the gene expression of a specific disease-related gene family (particularly, the disease-related GPCR gene family of which genes are found in some patients), one can determine which gene among many is responsible for the characteristic gene expression in a single operation.

Therefore, a medicine comprising an agonist, antagonist, antibody against the gene product of the specified gene, or a DNA coding for the gene product may be particularly effective for patients that are the subject of such a diagnosis. The present invention makes it possible to specify plural abnormally expressed genes and even to quantify with accuracy the expression level of those genes. Therefore, it is possible to select plural agonists, antagonists, or antibodies as an adequate prescription for the patient and to adjust the amount of this prescription according to the patient's expression level of the disease-related gene. That is, the present invention allows preparation of a tailor-made medicine that will be prescribed specifically for the patient.

More specifically, for example, the following treatments may be effective in a patient whocannot expect a physiological function of a ligand to a certain receptor protein because of reduced levels of the receptor protein (as in the case of a patient with a deficiency syndrome of the receptor protein). It is possible to increase the amount of this receptor protein in the patient to ensure sufficient activities of the ligand by ① administering said receptor protein to the patient by prescribing a sufficient amount of the receptor protein, ② either (i) administering a DNA of the present invention coding for the needed receptor protein to the patient, and expressing the DNA, or (ii) implanting a cell within the patient after inserting a DNA coding for the receptor protein into the desired cell and expressing the DNA.

The medicine of the present invention is effective in the preservation or treatment of a disease related to a specified gene, for example, diseases of the central nervous (for example, Alzheimers disease, dementia, eating disorders, or the like), endocrinopathy (for example, hypertension, abnormal gonadal function, abnormal thyroid function, abnormal pituitary function, or the like), metabolic disease (for example, diabetes, lipidosis, hyperlipidemia or the like), cancer (for example, non-parvicellular lung cancer, ovarian cancer, prostate cancer, stomach cancer, urinary bladder cancer, breast cancer, cervical cancer, colon cancer, rectal cancer, or the like).

When a gene product of a gene specified by the present invention (for example, receptor protein), an agonist, antagonist or antibody thereto, or a DNA encoding the gene, is used as a prophylactic/therapeutic agent as mentioned above, a pharmaceutical preparation can be prepared in a conventional manner.

On the other hand, where the DNA encoding the protein of the present invention (hereinafter sometimes referred to as the DNA of the present invention) is used as a prophylactic/therapeutic agent as described above, the DNA itself is administered; alternatively, the DNA is inserted into an appropriate vector such as a retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.

For example, ① the medicine of the present invention can be used orally, for example, in the form of tablets which may be sugar coated if necessary, capsules, elixirs, microcapsules etc., or parenterally in the form of injectable preparations such as a sterile solution and a suspension in water or with another pharmaceutically acceptable liquid. These preparations can be manufactured by mixing ① the protein of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, Gaultheria adenothrix oil, and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to humans or mammals (e.g., rats, rabbits, sheep, swine, bovine, cats, dogs, monkeys, etc.).

The dose of the medicine of the present invention varies depending on the subject to which it will be administered, target organs, conditions, routes for administration, etc.; in oral administration, e.g., an adult patient, the dose is normally about 0.1 mg to about 100 mg, preferably about 1.0 to about 50 mg, and more preferably about 1.0 to about 20 mg per day (for 60 kg body weight). In parenteral administration, the single dose varies depending on the subject to which it will be administered, target organ, conditions, routes for administration, etc., but it is desirable, e.g., for an adult patient, to administer the active ingredient intravenously in a daily dose of about 0.01 to about 30 mg, preferably about 0.1 to about 20 mg, and more preferably about 0.1 to about 10 mg (for 60 kg body weight). For other animal species, the corresponding dose as converted per 60 kg body weight can be administered.

**(Denotation of Abbreviations)**

In the specification and drawings, the codes of bases, amino acids, compound and others are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | : Deoxyribonucleic acid |
| cDNA | : Complementary deoxyribonucleic acid |
| A | : Adenine |
| T | : Thymine |
| G | : Guanine |
| C | : Cytosine |
| RNA | : Ribonucleic acid |
| mRNA | : Messenger ribonucleic acid |

dATP : Deoxyadenosine triphosphate
dTTP : Deoxythymidine triphosphate
dGTP : Deoxyguanosine triphosphate
dCTP : Deoxycytidine triphosphate
ATP : Adenosine triphosphate
EDTA : Ethylenediamine tetraacetic acid
SDS : Sodium dodecyl sulfate
Gly : Glycine
Ala: Alanine
Val: Valine
Leu: Leucine
Ile: Isoleucine
Ser: Serine
Thr: Threonine
Cys: Cysteine
Met: Methionine
Glu : Glutamic acid
Asp : Aspartic acid
Lys : Lysine
Arg : Arginine
His : Histidine
Phe : Phenylalanine
Tyr : Tyrosine
Trp : Tryptophan
Pro : Proline
Asn : Asparagine
Gln : Glutamine
pGlu : Pyroglutamic acid
Tos : P-toluenesulfonyl
CHO : Formyl
Bzl : Benzyl
$Cl_2Bzl$ : 2,6-dichlorobenzyl
Bom : Benzyloxymethyl
Z : Benzyloxycarbonyl
Cl-Z : 2-chlorobenzyloxycarbonyl
Br-Z : 2-bromobenzyloxycarbonyl
Boc : T-butoxycarbonyl
DNP : Dinitrophenol
Trt : Trityl
Bum : T-butoxymethyl
Fmoc : N-9-fluorenylmethoxycarbonyl
HOBt : 1-hydroxybenztriazole
HOOBt : 3,4-dihydro-3-hydroxy-4-oxo-1,2,3-benzotriazine
HONB : 1-hydroxy-5-norbornene-2,3-dicarboximide
DCC : N,N'-dicyclohexylcarbodiimide

The sequence identification numbers in the sequence listing of the specification indicates the following sequence, respectively.

[SEQ ID NO:1]

This shows the base sequence of h MOR1.

[SEQ ID NO:2]

This shows the base sequence of an up stream primer N-917F used in Example 1.

[SEQ ID NO:3]

This shows the base sequence of a down stream primer N-998R used in Example 1.

[SEQ ID NO:4]

This shows the base sequence of probe N-945T used in Example 1.

[SEQ ID NO:5]

This shows the base sequence of primer for detection of corn CB351.

[SEQ ID NO:6]

This shows the base sequence of primer for detection of corn CB351.

[SEQ ID NO:7]

This shows the base sequence of primer for detection of papaya 55-1.

[SEQ ID NO:8]

This shows the base sequence of primer for detection of papaya 55-1.

[SEQ ID NO:9]

This shows the base sequence of primer for detection of potato New Leaf Y.

[SEQ ID NO:10]

This shows the base sequence of primer for detection of potato New Leaf Y.

**EXAMPLES**

The present invention is described in detail below with reference to EXAMPLES, which are not deemed to limit the scope of the present invention.

**EXAMPLE 1**

(1) Drawing up the calibration curve for chemically synthesized DNA encoding a partial sequence of hMOR1

This example describes drawing up the calibration curve for chemically synthesized DNA encoding a partial sequence of hMOR1 cDNA (SEQ ID NO:1 : GenBank accession number L25119) by TaqMan method.

(2) Samples

The amplification was carried out using serial dilution solution of the chemically synthesized DNA encoding a partial sequence of hMOR1 cDNA (hMOR1T/M). The sequence of hMORICOM of the present invention corresponds to the complementary sequence from the 1129th to the 1210$^{th}$ bp of hMOR1 cDNA. After synthesis by β-cyanoethylphosphoamidaide solid-synthesis method (chemical synthesis method), ammonia cleavage treatment was carried out, followed by purification by polyacrylamide modified gel electrophoresis.

(3) Amplification primers and detection probes

The amplification of hMOR1 cDNA partial sequence region was carried out by using up stream primer N-917F having the sequence 5'-CCTTGGTTACAATCCCAGAAACTAC-3' (SEQ ID NO:2), and down stream primer N-998R having the sequence 5'-GCAGCTGTTTGTGTAACCTAGA-3' as a pair of primers.

The above up stream primer, N-917F hybridizes the complementary sequence from the 1129th-1153th bp of hMOR1 cDNA (SEQ ID NO:1). The above down stream primer, N-998R hybridizes the sequence from the 1187th-1210th bp of hMOR1 cDNA (SEQ ID NO:1). These primers catalyze the amplification of an 82 base pair product comprising one part of the entire length of the hMOR1 cDNA sequence.

The detection was carried out by using N-945T, having 5'-CCAGACTGTTTCTTGGCACTTCTGCATTG-3' (SEQ ID NO:4) as a probe. This probe hybridizes to the complementary sequence from the 1157th -1185th bp of hMOR1 cDNA (SEQ ID NO:1).

In order to make it possible to carry out the detection with the TaqMan method, the above probe was labeled with a fluorescein type fluorescent dye (FAM: reporter) at the 5' terminal and a rhodamine type fluorescent dye (TAMRA: quencher) at the 3' terminal.

In the labeled probe, the fluorescence of the reporter is suppressed when it does not hybridize, because of a migration phenomenon of fluorescent resonant energy. To prevent extension of the above probe by DNA polymerase during amplification, the 3' terminal of the above probe is blocked with a phosphoric acid.

(4) Amplification

Each PCR amplification was carried out with a total reaction amount of 20 μl using TaqMan™ Universal PCR Master MIX (Applied Bio Systems Japan Co. Ltd,.). The following is the final reagent concentration : sample gene, 1×TaqMan™ Universal PCR Master MIX (comprising AmpliTaq Gold™ DNA polymerase, AmpErase™ Urasil-N-glycosylase (UNG), ROX and others), 900nM of each primer, 200nM of probe.

The amplification reaction was carried out with ABI PRISM (trade mark) 7900HT sequence detection system (Applied Bio Systems Japan Co. Ltd.). The following is a profile of the thermal cycle used.

The time and temperature of the thermal cycle and the incubation in Amp Erase UNG reaction : two minutes at 50 °C, activation of the AmpliTaq Gold DNA polymerase : ten minutes at 95°C, denaturation/annealing/extension : 40 cycles : 15 seconds at 95°C, 1 minute at 60°C.

(5) Quantitative TaqMan Analysis

In the TaqMan analysis, during amplification, the probe hybridizing to the above target sequence is hydrolyzed from the 5' terminal by 5'-3' exonuclease reaction by the above DNA polymerase. Then, the reporter fluorescent dye is released and the fluorescence intensity increases.

The stored amplified product was quantified by measuring the increase of fluorescent intensity of the reporter dye in the reaction solution. At the same time, the fluorescent intensity of the fluorescent reference (fluorescent dye: ROX) was also measured to determine experimental error in the reaction solution. During each amplification cycle, the above reporter fluorescent dye and reference dye excite at the wavelength of light near the greatest excitation. Emission of the above reporter fluorescent dye and reference fluorescent dye are measured at the time of the greatest emission. This frequency is determined by the ABI PRISM™ 7900HT sequence detection system in advance. If another detection instrument is used, then an adequate frequency should be selected.

The value of the fluorescence was analyzed by 7900HT SDS software (Applied Bio Systems Japan Co. Ltd.). First, the fluorescent intensity of the reporter fluorescent dye was standardized by a reference fluorescent dye, and then a standardized reporter signal (Rn) was calculated. Then, the value calculated as being the average value (baseline) of relatively constant Rn during each cycle of the PCR primary cycle was designated as $_{\Delta}$Rn. When the amplification curve was plotted, the cycle number which analysis algorithm detected an increase of $_{\Delta}$Rn to the number of cycles, an increase of fluorescent signal ($_{\Delta}$Rn) corresponding to an exponential amplification of the amplification product for the first time was designated as the Threshold Cycle ($C_T$). In particular, in order to determine the $C_T$, PCR primary cycles (3-15 cycles) that are not considered to have achieved exponential amplification of the amplification product were used as a baseline, and the standard deviation of an average $_{\Delta}$Rn in this cycle was calculated. Then, the value of this standard deviation was multiplied ten times and defined as the "Threshold". The cycle number corresponding to this Threshold value on each amplification curve was defined as $C_T$.

During the period of exponential amplification of the above amplification product, the $C_T$ is proportional to the logarithm of the first target copy number. Accumulation of the amplification product in the later stage cycles inhibits the reaction and at last leads to the plateau of the reaction.

(6) Results

The following are $C_T$ values obtained from each sample. Each $C_T$ represents the average obtained from four reactions.

| Sample | $C_T$ value |
|---|---|
| $10^7$ copies of hMOR1T/M | 16.3 |
| $10^6$ copies of hMOR1T/M | 19.2 |
| $10^5$ copies of hMOR1T/M | 22.5 |
| $10^4$ copies of hMOR1T/M | 26.0 |
| $10^3$ copies of hMOR1T/M | 29.2 |
| $10^2$ copies of hMOR1T/M | 32.9 |
| 0 copy of hMOR1T/M | above 40.0 |

The calibration curve was prepared using the $C_T$ value obtained from the amplification of a known amount of template hMOR1COM (standard sample). In particular, the calibration curve was drawn up by plotting $C_T$ against this known primary amount of standard sample (logarithm value). For an approximated curve, the following linear equation was used.

$$C_T = (\text{Log }[DNA]_T - \text{Log}[DNA]_o)/\text{Log}(1+e)$$

(wherein $[DNA]_o$ is a primary concentration, $[DNA]_T$ is a concentration of an amplification product at a $C_T$ cycle, e is an average amplification efficiency, and Log(X) is a logarithm where X represents a base of log 10). The 7900HT SDS software was used to to determine parameters.

By using the value of $C_T$ obtained from samples, the following calibration curve was obtained:

$$C_T = 39.34\text{-}3.331 \times \text{Log }[DNA]_0$$

Correlation coefficient R2 = 0.999
Average amplification efficiency e = 99.6%

**EXAMPLE 2**

(1) Extraction of RNA and synthesis of DNA

Prostate cancer cells (LNCaP-FGC cells) were cultured until preconfluent. After removing the cells with 0.25% trypsin-1mM EDTA (Invitrogen Co. Ltd.) and counting the number of cells, total RNA was extracted and purified according to the manual instructions of the RNeasy mini KIT (QUAGEN Co. Ltd.). The first strand cDNA was synthesized from the extracted RNA according to the manual instructions of the Superscript II (Invitrogen Co. Ltd.), and after ethanol precipitation, the cDNA was eluted and used as below. Synthesized cDNA was dissolved in TE to correspond to 10 mg/ml RNA and diluted to correspond to 5 ng/µl in TE comprising 50 µg/ml yeast tRNA. Five (5) µl of diluted cDNA solution (corresponding to 25 ng of RNA) was used as a measuring sample for a quantification of mRNA of one kind of GPCR.

(2) Quantification of GPCR mRNA using chemical synthesized standard preparation

Based upon the known sequences of 160 kinds of GPCRs, primers and probes were designed using Primer Express™software (Applied Bio Systems Japan Co. Ltd.). Further, (-) strand DNA having a sequence between the primers was chemically synthesized. After diluting chemically synthesized DNA to $10^6$ copies / 5µl with TW comprising 50 µg/ml yeast tRNA, dilution series were prepared until a concentration of $10^2$ copies / 5µl each were achieved at 10x. The measurement of one kind of GCPR mRNA was carried out by dispensing 5µ of each of five kinds of dilution series of those chemically synthesized products and one of the above measuring samples by duplex. As the amplification reagents, TaqMan™ Universal PCR Master kit (Applied Bio Systems Japan Co. Ltd.) and the above designed TaqMan™ Probe Kit (Applied Bio Systems Japan Co. Ltd.) were used. After preparing an amount of 15µl solution of the amplification reagents, those solutions were added to each well containing the above standard preparation or the measuring sample. The final concentration of each primer and probe was adjusted according to manual instructions. TaqMan™ PCR was carried out by ABI PRISM™ 7900HT sequence detection system (Applied Bio Systems Japan Co. Ltd.), using the thermal cycles described in the manual of TaqMan™ universal PCR Master Mix (Applied Bio Systems Japan Co. Ltd.)

The quantitative TaqMan analysis of the amplification product was carried out by 7900HT SDS software (Applied Bio Systems Japan Co. Ltd.). Using the above conditions, quantitative measurement of the 32 kinds of GPCR was carried out in one well of a 384-well plate. Use of five plates of a 384-well plate allowed accurate quantification of all the aforementioned 160 GPCRs expressed in the prostate cancer cell strain (LNCaP-FGC cells).

## Industrial Applicability

The standard preparation of the present invention is a synthetic polynucleotide with the advantage that a desired sequence can accurately be obtained by a concise method compared to the standard preparation consisting of a polynucleotide obtained by conventional biosynthesis. Further, the standard preparation of the present invention is not biologically contaminated. Thus, the standard preparation of the present invention is safe for the environment, and has the additional advantage of reducing factors that inhibit highly accurate quantification.

Moreover, when a single strand polynucleotide is used as the standard preparation, the quantitative method and the quantitative kit of the present invention allow more accurate quantification because of similarity of the standard used to substance to be quantified.

Furthermore, the other embodiment of the quantitative method and the quantitative kit of the present invention can, with a single manipulation, detect with high-sensitivity an amount of the multiple target nucleic acids in samples that may contain multiple target nucleic acids. Therefore, the present invention provides a system that can carry out expression analysis of the target nucleic acid with high-sensitivity.

Furthermore, the quantification method and quantification kit of the present invention make it possible to diagnose specific diseases and examine genetically modified foods to determine if recombinant DNA is present.

**Claims**

1. A standard preparation useful to quantify or detect a specific target nucleic acid in a sample, comprising a synthetic polynucleotide obtained by chemical synthesis.

2. The standard preparation of claim 1, wherein the synthetic polynucleotide is RNA, DNA or a modification thereof.

3. The standard preparation of claim 1, wherein the synthetic polynucleotide is RNA or DNA.

4. The standard preparation of claim 3, which is a sense strand if the synthetic polynucleotide is RNA, or which is an antisense strand if the synthetic polynucleotide is DNA.

5. The standard preparation of claim 1, wherein the synthetic polynucleotide is a synthesized part of the target nucleic acid, and the number of nucleotides is between 60 and 200.

6. A kit for quantifying nucleic acid comprising the standard preparation of any one of claims 1 to 5.

7. A kit for quantifying nucleic acid comprising the standard preparation of any one of claims 1 to 5 and at least one pair of primers.

8. The kit of claim 7, additionally comprising a fluorescence probe or a phosphorylated probe.

9. The kit of claim 8, additionally comprising a DNA polymerase.

10. The kit of claim 9, additionally comprising a reverse transferase.

11. A kit for quantifying nucleic acid to quantify multiple target nucleic acids, wherein an amplification reagent comprising a pair of primers corresponding to the target nucleic acid is loaded at each reaction site of a reactor having multiple reaction sites, and an amplification reagent comprising the standard preparation of any one of claims 1 to 5 and a pair of primers corresponding to the standard preparation is loaded at a reaction site which is not loaded with a pair of primers corresponding to the target nucleic acid.

12. The kit for quantifying nucleic acid of claim 11, which is used to diagnose a specific disease, and wherein the multiple target nucleic acids are DNA or mRNA related to the specific disease.

**13.** The kit for quantifying nucleic acid of claim 11, which is used to detect recombinant DNA in food, and wherein the multiple target nucleic acids are recombinant DNA contained in genetically-modified food.

**14.** A method for quantifying a specific target nucleic acid in a sample, which comprises adding an amplification reagent comprising at least one pair of primers corresponding to a target nucleic acid to the sample, adding, to a chemically synthesized polynucleotide as a standard preparation, an amplification reagent comprising a pair of primers corresponding to the synthesized polynucleotide, carrying out each amplification reaction, measuring the amounts of the amplified standard preparation and the amplified target nucleic acid, and calculating the amount of the target nucleic acid before amplification according to the information obtained by the measurements.

**15.** The method of claim 14, wherein the synthetic polynucleotide is RNA, DNA or a modification thereof.

**16.** The method of claim 14, wherein the synthetic polynucleotide is RNA.

**17.** The method of claim 16, wherein the synthetic polynucleotide is a sense strand.

**18.** The method of any one of claims 14 to 17, wherein the synthetic polynucleotide is a synthesized part of the target nucleic acid, and the number of nucleotides is between 60 and 200.

**19.** The method of any one of claims 14 to 18, wherein the sample is an mRNA sample of human or other animal origins.

**20.** The method of claim 19, wherein the amplification reagent additionally comprises a fluorescence probe or a phosphorylated probe.

**21.** The method of claim 20, wherein the amplification reagent additionally comprises a DNA polymerase.

**22.** The method of claim 21, wherein the amplification reagent additionally comprises a reverse transferase.

**23.** The method of any one of claims 20 to 22, wherein (1) a probe portion of a fluorescence probe or a phosphorylated probe contained in the amplification reagent comprising at least a pair of primers corresponding to a target nucleic acid, is a probe consisting of the nucleic acid region between the pair of primers in the target nucleic acid, and (2) a probe portion of a fluorescence probe or a phosphorylated probe contained in the amplification reagent comprising the pair of primers corresponding to the synthetic polynucleotide, is a probe consisting of the nucleic acid region between the pair of primers in the synthetic polynucleotide.

**24.** The method of claim 23, which comprises measuring the amount of the amplified standard preparation and the amount of the amplified target nucleic acid using the fluorescence intensity of the fluorescent substance released from the fluorescence probe or the phosphorylated probe by DNA polymerase or the amount of phosphate group as an index.

**25.** A method for analyzing SNPs, which uses the kit of claim 11 or the method of claim 14.

**26.** A method for diagnosing a specific disease, which uses the kit of claim 12 or the method of claim 14.

**27.** A method for determining if food contains recombinant gene DNA or not, which uses the kit of claim 13 or the method of claim 14.

**28.** A medicine specified by the kit of claim 12 or the method of claim 26, which comprises a gene DNA in which expression is distinctively increased or reduced in a certain cell or tissue, a gene product thereof, or an agonist, antagonist or antibody against the gene product.

**INTERNATIONAL SEARCH REPORT**

International application No.
PCT/JP02/13782

A. CLASSIFICATION OF SUBJECT MATTER
Int.Cl[7] C12N15/09, C12Q1/68, G01N33/566, A61K45/00, A61K39/395, A61K48/00, A61P43/00

According to International Patent Classification (IPC) or to both national classification and IPC

B. FIELDS SEARCHED

Minimum documentation searched (classification system followed by classification symbols)
Int.Cl[7] C12N15/09, C12Q1/68, G01N33/566, A61K45/00, A61K39/395, A61K48/00, A61P43/00

Documentation searched other than minimum documentation to the extent that such documents are included in the fields searched

Electronic data base consulted during the international search (name of data base and, where practicable, search terms used)
BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
|---|---|---|
| X | WO 01/46463 A2 (BAXTER AG.), 28 June, 2001 (28.06.01), & US 2002/102548 A1 & EP 1244814 A2 | 1-27 |
| A | JP 2001-95576 A (Shimadzu Corp.), 10 April, 2001 (10.04.01), (Family: none) | 1-27 |
| A | JP 11-9281 A (Shimadzu Corp.), 19 January, 1999 (19.01.99), (Family: none) | 1-27 |
| A | WO 91/02817 A (Rosh), 07 March, 1991 (07.03.91), & US 5219727 A & EP 497784 A & JP 5-504886 A | 1-27 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 01 April, 2003 (01.04.03) | 15 April, 2003 (15.04.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13782

**Box I Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 28
because they relate to parts of the international application that do not comply with the prescribed requirements to such an extent that no meaningful international search can be carried out, specifically:
Concerning "a drug containing a gene DNA, a gene product, an agonist, an antagonist or an antibody" as set forth in claim 28, no specific compound contained in the drug but merely general statement is given in the description. Thus, it is unknown what specific substances are (continued to extra sheet)

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)

**INTERNATIONAL SEARCH REPORT**

International application No.
PCT/JP02/13782

Continuation of Box No.I-2 of continuation of first sheet(1)

contained in the above drug. Such being the case, no meaningful international search can be made on claim 28.

Form PCT/ISA/210 (extra sheet) (July 1998)